Europäisches Patentamt

European Patent Office

Office européen des brevets

(11) Veröffentlichungsnummer: **0 187 732**
**A2**

(12) # EUROPÄISCHE PATENTANMELDUNG

(21) Anmeldenummer: 86810001.7

(22) Anmeldetag: 06.01.86

(51) Int. Cl.⁴: **C 12 N 1/20**
**C 12 N 1/32, C 02 F 3/34**
**C 05 F 11/08**
**//(C12N1/20, C12R1:01)**

(30) Priorität: 09.01.85 CH 75/85

(43) Veröffentlichungstag der Anmeldung:
16.07.86 Patentblatt 86/29

(84) Benannte Vertragsstaaten:
AT BE CH DE FR GB IT LI LU NL SE

(71) Anmelder: CIBA-GEIGY AG
Klybeckstrasse 141
CH-4002 Basel(CH)

(72) Erfinder: Ghisalba, Oreste, Dr.
Feldbergstrasse 91
CH-4057 Basel(CH)

(72) Erfinder: Ramos, Gerardo, Dr.
General Guisan-Strasse 35
CH-4144 Arlesheim(CH)

(72) Erfinder: Schär, Hans-Peter, Dr.
Eggfluhweg 11
CH-4147 Aesch(CH)

(72) Erfinder: Küenzi, Martin, Dr.
Hüslimattstrasse 48
CH-4132 Muttenz(CH)

(54) Mikroorganismen des Genus Hyphomicrobium und Verfahren zum Abbau von Methylgruppen-haltigen Verbindungen, insbesondere von Methylgruppen-haltigen Phosphorverbindungen, in wässrigen Lösungen.

(57) Die vorliegende Erfindung betrifft neue fakultativ methylotrophe Mikroorganismen des Genus Hyphomicrobium, proteinhaltige Zellmasse, sowie ein Verfahren zur mikrobiologischen Reinigung von wässrigen Lösungen, z.B. Abwässern, welche als Verunreinigungen insbesondere Trimethylphosphat, Dimethyl- oder Methylphosphatsalze, Dimethylmethanphosphonat oder Methylmethanphosphonatsalze enthalten. Man züchtet den jeweiligen Mikroorganismus in wässriger Phase und baut dabei die Verunreinigungen ab.

EP 0 187 732 A2

Croydon Printing Company Ltd.

CIBA-GEIGY AG                                    64-15217/=

Basel (Schweiz)

Mikroorganismen des Genus Hyphomicrobium und Verfahren zum Abbau von
Methylgruppen-haltigen Verbindungen, insbesondere von Methylgruppen-haltigen Phosphorverbindungen, in wässrigen Lösungen

Die vorliegende Erfindung betrifft neue fakultativ methylotrophe
Mikroorganismen des Genus Hyphomicrobium, ein Verfahren zum mikrobiologischen Abbau von Methylgruppen-haltigen Verbindungen, insbesondere von Methylgruppen-haltigen Phosphorverbindungen in wässrigen
Phasen in Gegenwart dieser Mikroorganismen, die Zellmasse mit ihren
Bestandteilen, welche von diesen Mikroorganismen hergestellt wird,
sowie die Verwendung der verfahrensgemäss erhältlichen Zellmasse.

Unter dem Begriff "methylotrophe Mikroorganismen" versteht man
solche Mikroorganismen, die auf Nährmedien wachsen, welche als
Kohlenstoffquelle Verbindungen mit nur einem Kohlenstoffatom, z.B.
Methanol, oder mehreren Kohlenstoffatomen ohne direkte C-C-Bindung,
z.B. Dimethylamin, enthalten [Patt, Cole und Hansen, International
J. Systematic Bacteriology 26 (2), 226-229 (1979)].

Unter dem Begriff "fakultativ methylotrophe Mikroorganismen"
versteht man solche Mikroorganismen, die auf Nährmedien wachsen,
welche als Kohlenstoffquelle Verbindungen mit nur einem Kohlenstoffatom oder mehreren Kohlenstoffatomen ohne direkte C-C-Bindung
und/oder Verbindungen mit mehreren Kohlenstoffatomen mit C-C-
Bindung, z.B. Aethanol, enthalten.

In der Literatur sind fakultativ methylotrophe Mikroorganismen beschrieben, welche in wässriger Lösung eine oder mehrere Verbindungen aus der folgenden Gruppe von organischen Verbindungen abbauen bzw. als Kohlenstoff- oder als Kohlenstoff- und Stickstoffquelle verwerten können: Methan, Methanol, Aethanol, Acetate, z.B. Natriumacetat, Glucose, bestimmte Alkylammoniumverbindungen, z.B. Methyl-, Aethyl- oder Trimethylammoniumchlorid, oder die freien Amine von diesen Salzen, Methylsulfat, z.B. Natriummethylsulfat, oder Dimethyl- oder Trimethylphosphit.

Solche Mikroorganismen sind in verschiedenen Stammsammlungen, z.B. in der Agricultural Research Culture Collection (NRRL), in der American Type Culture Collection (ATCC), in der Deutschen Sammlung von Mikroorganismen (DSM) oder in der National Collection of Industrial Bacteria (NCIB), hinterlegt. Einige Hinterlegungsstellen publizieren Kataloge mit den dort hinterlegten Mikroorganismen.

In verschiedenen Publikationen sind Mikroorganismen unterschiedlicher Genera, z.B. Pseudomonas, Klebsiella oder Escherichia coli erwähnt, welche Phosphor-Kohlenstoff-Verbindungen, z.B. Methylphosphate oder Methylphosphonate, abbauen können, z.B. Cook A. M. et al.: Phosphonate Utilization by Bacteria, J. Bacteriol. 133 (1978) 85-90. Einige Methylphosphat- oder Methylphosphonat-abbauende Stämme sind in der Deutschen Sammlung für Mikroorganismen (DSM) hinterlegt, z.B. Klebsiella pneumoniae DSM 1620, Pseudomonas acidovorans DSM 1621 oder Pseudomonas testosteroni DSM 1622. Diese Mikroorganismen haben alle den Nachteil, dass sie nur geringe Mengen an Methylphosphaten oder Methylphosphonaten, z.B. Dimethylmethanphosphonat, Methanphosphonat, Dimethylphosphat oder Monomethylphosphat, abbauen und diese Verbindungen auch nur als Phosphorquelle aber nicht als Kohlenstoffquelle verwerten können. Beim Abbau dieser Verbindungen muss ausserdem eine Kohlenstoffquelle zugesetzt werden und zwar in weitaus grösseren Mengen als es den Mengen eingesetzter Phosphor-Verbindung entspricht (Molverhältnis C-Quelle zu P-Quelle ca. 30:1-50:1).In die gebildete Biomasse können nur geringe Mengen Phosphor eingebaut werden, so dass für ein Mol Phosphor (31 g) ca. 1,0 kg Trockenbiomasse entstehen, siehe Cook et al., Appl. Environm.
Microbiol. 36, 1978, S. 670. Gewisse Methylphosphate und Methylphosphonate werden von den bekannten, in der Literatur beschriebenen
Mikroorganismen überhaupt nicht abgebaut, z.B. Trimethylphosphat
oder Monomethylmethanphosphonat.

In der Literatur gibt es keine Angaben über den Fundort, die
Isolierung, Identifizierung oder die Hinterlegungsstelle von
Mikroorganismen, welche Methylphosphate oder Methylphosphonate als
Kohlenstoffquelle verwerten können. Ebenso fehlt in der Literatur
ein Hinweis, Mikroorganismen in einem mikrobiologischen Verfahren
zur Reinigung von Abwasser enthaltend Methylphosphate oder Methylphosphonate zu verwenden.

Bei der grosstechnischen Produktion in der chemischen Industrie
fallen wässrige Lösungen, z.B. Abwässer, an, welche beispielsweise
Phosphorsäuremethylester, Mono- oder Diäthylphosphat oder Methanphosphonsäuremethylester und Salze dieser Verbindungen als Verunreinigungen enthalten. Die Beseitigung solcher Abwässer mit den
bisher üblichen Verbrennungsverfahren ist äusserst ungünstig, denn
die Verbrennung von organischen Abfallstoffen gilt als problematische Beseitigungsmethode wegen hoher Kosten und ebenfalls grosser
Belastungen für die Umwelt.

Der vorliegenden Erfindung liegt die Aufgabe zugrunde, neue Mikroorganismen zu finden und zu isolieren, welche in wässrigen Lösungen
unter möglichst vollständigem Verbrauch der genannten Verbindungen
und Salze als C-Quelle sowie weiterer Verunreinigungen, z.B.
Methyl-, Dimethyl-, Trimethyl- oder Aethylammoniumsalze oder
Formiat- oder Acetatsalze sowie Methanol oder Aethanol, ein besonders gleichmässiges und rasches Wachstum zeigen.

Diese Aufgabe wird durch die vorliegende Erfindung gelöst, welche
neue fakultativ methylotrophe Mikroorganismen des Genus Hyphomikrobium, Mischkulturen und Mutanten davon zum Gegenstand hat. Die vorliegende Erfindung betrifft ebenfalls ein Verfahren zum
mikrobiologischen Abbau von Phosphorsäuremethylestern, Mono- oder
Diäthylphosphat, Methanphosphonsäuremethylestern und Salzen dieser
Verbindungen, Methyl-, Dimethyl-, Trimethyl- oder Aethylammoniumsalzen oder Formiat- oder Acetatsalzen sowie von Methanol oder
Aethanol oder Mischungen dieser Verbindungen oder Salze in wässrigen
Phasen unter Verwendung dieser neuen Mikroorganismen, die Zellmasse
mit ihren Bestandteilen, welche von diesen neuen Mikroorganismen
oder von Mischkulturen dieser Mikroorganismen hergestellt wird,
sowie die Verwendung der verfahrensgemäss erhältlichen Zellmasse.

Die weiter vorn und im folgenden verwendeten allgemeinen Begriffe
haben in der Beschreibung der vorliegenden Erfindung vorzugsweise
folgende Bedeutungen:

Phosphorsäuremethylester sind z.B. Trimethyl-, Dimethylhydrogen-
oder Dihydrogenmethylphosphat.

Methanphosphonsäuremethylester sind Dimethylmethanphosphonat oder
Hydrogenmethylmethanphosphonat.

Salze sind insbesondere die Natrium-, ferner die Kalium- oder
Lithiumsalze.

Salze der genannten Phosphorsäuremethylester, von Mono- oder
Diäthylphosphat oder von Methanphosphonsäuremethylestern sind insbesondere Natriumdimethylphosphat, Natriumhydrogenmethylphosphat,
Dinatriummethylphosphat, Natriumdiäthylphosphat oder Natriummethylmethanphosphonat.

Die genannten Methyl-, Dimethyl-, Trimethyl- oder Aethylammoniumsalze sind vorzugsweise die Hydrohalogenide der freien Amine,
vorzugsweise die Hydrochloride.

Die genannten Formiat- oder Acetatsalze sind insbesondere die
Natrium-, ferner die Kalium- oder Lithiumsalze.

Zellmasse ist die Gesamtmenge aller sich in lebendem Zustand, z.B. Teilungs- oder Ruhezustand, oder in abgestorbenem Zustand befindlichen Zellen oder Zellbestandteile, die aus der wässrigen Phase abtrennbar sind, z.B. durch Filtration, Zentrifugation oder Sedimentation.

Bestandteile dieser Zellmasse sind z.B. die darin enthaltenen Enzyme bzw. die aus der Zellmasse erhältlichen Enzymextrakte, welche ebenfalls die weiter vorn genannten Verbindungen bzw. Verunreinigungen, z.B. Dimethylphosphat, in wässriger Lösung abbauen können.

Die vorliegende Erfindung hat in erster Linie neue, fakultativ methylotrophe Mikroorganismen des Genus Hyphomicrobium aus der Gruppe folgender Stämme: TMPO 1/8 (NRRL-B-15632), TMPO 1/9 (NRRL-B-15633), TMPO 1/10, (NRRL-B-15634), TMPO 2/3 (NRRL-B-15635), TMPO 2/6 (NRRL-B-15636), TMPO 2/9 (NRRL-B-15637), TMPO 2/10 (NRRL-B-15638), TMPO 2/13 (NRRL-B-15639), DMMP 1/6 (NRRL-B-15640), DMMP 1/9 (NRRL-B-15641), DMMP 2/10 (NRRL-B-15642), DMMP 2/11 (NRRL-B-15643), DMMP 2/15 (NRRL-B-15644) und DMPO/HW1-5 (NRRL-B-15645), Mischkulturen und Mutanten davon zum Gegenstand.

Die neuen Mikroorganismen stammen aus dem Klärschlamm einer Abwasserreinigungsanlage der Ciba-Geigy AG Schweiz (ARA Ciba-Geigy) und aus dem Klärschlamm eines Schlammteichs (Trockenbeet) in Kairouan, Tunesien. Die Kultur DMPO/HW1-5 wurde aus dem Sediment des Hallwilersees in der Schweiz (Probenentnahme bei Tennwil) isoliert. Sämtliche Mikroorganismen sind bei der Agricultural Research Culture Collection (NRRL) in Peoria, Illinois 61 604, USA, am 29.9.83 nach den Vorschriften des "Budapest Treaty on the International Recognition of the Deposit of Microorganisms for the Purposes of Patent Procedure" hinterlegt worden.

In der folgenden Tabelle 1 sind Hinterlegungsnummern, und Fundort für jeden einzelnen Stamm angegeben:

Tabelle 1:

| Interne Bezeichnung | Hinterlegungsnummer | Fundort |
|---|---|---|
| TMPO 1/8 | NRRL-B-15632 | ARA Ciba-Geigy |
| TMPO 1/9 | NRRL-B-15633 | ARA Ciba-Geigy |
| TMPO 1/10 | NRRL-B-15634 | ARA Ciba-Geigy |
| TMPO 2/3 | NRRL-B-15635 | Kairouan |
| TMPO 2/6 | NRRL-B-15636 | Kairouan |
| TMPO 2/9 | NRRL-B-15637 | Kairouan |
| TMPO 2/10 | NRRL-B-15638 | Kairouan |
| TMPO 2/13 | NRRL-B-15639 | Kairouan |
| DMMP 1/6 | NRRL-B-15640 | Kairouan |
| DMMP 1/9 | NRRL-B-15641 | Kairouan |
| DMMP 2/10 | NRRL-B-15642 | Kairouan |
| DMMP 2/11 | NRRL-B-15643 | Kairouan |
| DMMP 2/15 | NRRL-B-15644 | Kairouan |
| DMPO/HW1-5 | NRRL-B-15645 | Hallwilersee |

## Charakterisierung der neuen Mikroorganismen:

1. Isolierungsmethode:

Eine Abwasserprobe oder Klärschlammsuspension (ca. 1 g pro 10 ml
sterilem Wasser) wurde in einen Schüttelkolben gegeben, worin sich
20 ml sterile Nährlösung MV 7 befand. Man gab ca. 0,1 g sterilfiltriertes Trimethylphosphat (TMPO-Stämme NRRL-B-15632-15639),
Dimethylmethanphosphat (DMMP-Stämme NRRL-B-15640-15644) oder
Dimethylphosphat (DMPO-Stamm NRRL-B-15645) hinzu, versetzte zur
Pufferung der Nährlösung mit 1 ml 1 M Phosphatpuffer von pH 7 und
inkubierte bei 28°C die Schüttelkultur bei 250 Upm 7-14 Tage lang.
1 ml dieser ersten Anreicherungskultur wurde in 20 ml frische
Nährlösung MV 7 mit 0,1 g Trimethylphosphat (TMPO-Stämme), Dimethylmethanphosphat (DMMP-Stämme) bzw. Dimethylphosphat (DMPO-Stamm)
gegeben und nochmals bei 28° und pH 7 (Schüttelkultur 250 Upm, 7-14
Tage) inkubiert. 1 ml der zweiten Anreicherungskultur wurde wieder
in 20 ml frische Nährlösung MV 7 mit 0,1 g Trimethylphosphat
(TMPO-Stämme), Dimethylmethanphosphat (DMMP-Stämme) bzw. Dimethylphosphat (DMPO-Stamm) gegeben und unter den genannten Bedingungen
inkubiert. Aus 1 ml der dritten Anreicherungskultur stellte man
nochmals mit 20 ml Nährlösung MV 7 und 0,1 g Trimethylphosphat
(TMPO-Stämme), Dimethylmethanphosphat (DMMP-Stämme) bzw. Dimethyl-

phosphat (DMPO-Stamm) unter den genannten Bedingungen eine vierte Anreicherungskultur her. Die so hergestellten Anreicherungskulturen wurden nach kurzer Behandlung mit Ultraschall jeweils auf sterile feste MV 7-Agar Nährböden [Nährlösung MV 7 enthaltend 1 g/l Trimethylphosphat, Dimethylmethanphosphat bzw. Dimethylphosphat mit Zusatz von 20 g/l Agar (Difco)] plattiert und bei 28°C inkubiert. Einzelkolonien wurden vorsichtig abgehoben und wieder auf dem gleichen Medium ausgestrichen. Diese Prozedur wurde mehrmals wiederholt, bis reine Isolate vorlagen.

Die verwendete Nährlösung MV 7 enthält in einem Liter Wasser die folgenden Bestandteile: 2 g $NH_4NO_3$ (Stickstoffquelle), 1,4 g $Na_2HPO_4$, 0,6 g $KH_2PO_4$ (Puffer), 0,2 g $MgSO_4 \cdot H_2O$, 0,01 g $CaCl_2 \cdot 2\ H_2O$, 0,001 g $FeSO_4 \cdot 7\ H_2O$ und 1 ml Spurenelementlösung (bestehend aus je 20 mg/l $Na_2MoO_4 \cdot 2\ H_2O$, $Na_2B_4O_7 \cdot 10\ H_2O$, $MnSO_4 \cdot H_2O$ und $CuSO_4 \cdot 5\ H_2O$). Zur Herstellung dieser Nährlösung löst man die Salze in destilliertem Wasser, bringt die Lösung mit verdünnter, wässriger Natriumhydroxid-Lösung unter pH-Kontrolle auf pH 7 und füllt diese mit destilliertem Wasser auf 1 Liter auf. Zur Herstellung des festen Nährbodens MV 7-Agar werden der Nährlösung MV 7 noch 20 g/l Agar (Difco) zugesetzt. Man sterilisiert im Autoklaven (2 at, 120°C).

## 2. Allgemeine Befunde und Mikroskopie

Alle in der Tabelle 1 aufgezählten Stämme wachsen unter aeroben Bedingungen bei Temperaturen bis ca. 37°, optimal bei 28° bis 30°. Die Mikroorganismen sind gramnegativ (Proben für Gram-Färbetest aus einer MV 7-Dimethylphosphat-Kultur entnommen) und Oxidase-positiv (Proben für Test von Nutrient-Agar, MV 7-Dimethylphosphat-Agar und MV 7-Dimethylphosphat-Flüssigkultur entnommen). In flüssigen Kulturen, welche Dimethylphosphat oder Trimethylphosphat enthalten, beobachtet man eine Neigung aller Stämme zu flockigem Wachstum.

In der mikroskopischen Abbildung (Lichtmikroskop) sind sich alle Stämme sehr ähnlich und als kurze, z.T. motile Stäbchen von ca. 1-2 µ Länge mit filamentösen Auswüchsen (Hyphen) zu erkennen,

- 8 -                                    0187732

welche einzeln, paarweise oder agglomeriert auftreten. Das
elektronenmikroskopische Bild befindet sich in guter Uebereinstimmung mit den lichtmikroskopischen Beobachtungen. Ein Teil der
Zellen zeigt jeweils lange "Stiele" oder "Hyphen", wobei jede Zelle
einen, maximal zwei (selten) Stiele in polarer Position
besitzt. Einige gestielte Zellen tragen Geisseln. Bei einzelnen
gestielten Zellen ist am Ende des Stiels eine Verdickung (Knospe) zu
erkennen. Ein anderer Zelltyp besitzt zwar keinen Stiel, dafür aber
eine subpolare Geissel. Vereinzelt findet man auch verzweigte
Hyphen.

3. <u>Biochemische Charakterisierung und Klassifizierung der neuen
   Mikroorganismen</u>
Zur allgemeinen biochemischen Charakterisierung und Klassifizierung
der in Tabelle 1 aufgezählten Stämme werden zwei käufliche Testsysteme verwendet.

a) <u>"Oxi/Ferm Tube" - Test (Roche)</u>
Dieses Testsystem eignet sich für grammegative, Stäbchen-förmige
Bakterien mit positiver Oxidasereaktion. Der jeweilige Test wird
parallel je zweimal mit Impfmaterial des betreffenden Stamms
abgenommen von Dimethylphosphat - Agar und Nutrient - Agar durchgeführt. Beschreibung der genormten biochemischen Tests und experimentelle Ausführung siehe Vorschrift des Herstellers. Die Testresultate
sind für alle Stämme weitgehend identisch:

Tabelle 2:

| Biochemischer Test | Resultat |
|---|---|
| Anaerobe Dextrose-spaltung | negativ |
| Arginindihydro-lase | negativ |
| $N_2$-Produktion | nicht eindeutig |
| $H_2S$-Bildung | negativ |
| Indol-Bildung | negativ |
| Xylosespaltung | schwach positiv |
| aerobe Dextrose-spaltung | negativ |
| Urease | positiv |
| Citratverwertung | schwach positiv |

b) "API 20 E"-Test (Fa. Api, Genève)

Dieses Testsystem wird speziell zur Erkennung von Enterobacteriaceen und allgemein zur Bestimmung von gramnegativen Bakterien verwendet. Die genormten biochemischen Tests und ihre Ausführung sind in der Anleitung des Herstellers beschrieben. Die Tests wurden ebenfalls paralell je zweimal mit Impfmaterial des betreffenden Stamms abgenommen von Dimethylphosphat-Agar und Nutrient-Agar durchgeführt. Die Testresultate sind für alle Stämme weitgehend identisch:

0187732

Tabelle 3:

| Biochemischer Test | Resultat |
|---|---|
| ONPG: Hydrolyse durch ß-Galactosidase | schwach positiv |
| ADH: Arginindihydrolase | negativ |
| LDC: Lysindecarboxylase | negativ |
| ODC: Ornithindecarboxylase | negativ |
| CIT: Citratverwertung | positiv |
| H₂S: Thiosulfatspaltung | negativ |
| URE: Urease | schwach positiv |
| TDA: Tryptophandesaminase | negativ |
| IND: Tryptophanabbau Indolbildung | negativ |
| VP: Acetointest | positiv |
| GEL: Gelatineverflüssigung | nicht eindeutig |
| GLU: Glucoseverwertung | negativ |
| MAN: Mannitverwertung | negativ |
| INO: Inositverwertung | negativ |
| SOR: Sorbitverwertung | negativ |
| RHA: Rhamnoseverwertung | negativ |
| SAC: Saccharoseverwertung | negativ |
| MEL: Melibioseverwertung | negativ |
| AMY: Amygdalinverwertung | negativ |
| ARA: L-Arabinoseverwertung | negativ |
| OX: Oxidase | positiv |
| NO₂: / N₂: Nitratreduktion | positiv |
| CAT: Katalase | positiv |

Die Auswertung der mikroskopischen Befunde und der Resultate der
biochemischen Tests erlaubt die taxonomische Zuordnung der erfindungsgemässen Mikroorganismen zum Genus Hyphomicrobium.

4. Konservierung der Stämme

Für die Konservierung der Stämme gemäss Tabelle 1 eignen sich
folgende Methoden:

a) Adsorption des Zellmaterials des betreffenden Stamms auf Glaskügelchen in Glycerinlösung und anschliessende Lagerung bei -20°C,

b) Aufbewahrung des Zellmaterials des betreffenden Stamms über
Schrägagar und

c) Lyo-Ampullen. Die betreffende Kultur wird von der Nährlösung
abzentrifugiert und die Zellmasse in 1/4 bis 1/3 Volumenteilen
15 %iger Skim-Milk resuspendiert und lyophilisiert.

5. Abbaubare Verbindungen

Die Abbaubarkeit sämtlicher weiter vorn genannter Verbindungen und
Salze durch jeden Mikroorganismus gemäss Tabelle 1 wurde experimentell nach Herstellen einer Schüttelkultur durch Impfen einer
Trimethylphosphat-, Dimethylmethanphosphat- oder Dimethylphosphathaltigen Vorkultur in Nährlösung MV 7 enthaltend die betreffenden
abzubauenden Verbindungen C- bzw. C- und N-Quellen), Abpuffern auf
pH 7, Inkubation innerhalb von 7-30 Tagen bei 28°C und 250 Upm und
Messung der optischen Dichte $OD_{650}$ nachgewiesen. Dabei erfolgte in
jedem Fall vollständiger (mindestens 95 %) Abbau der betreffenden
Verbindung und Salze.

Von den in der Tabelle 1 aufgezählten Stämmen lassen sich durch
Vermischen von Vorkulturen Mischkulturen herstellen. Ebenso können
sich spontan Mutanten (natürliche Mutanten) bilden, oder es lassen
sich künstliche Mutanten herstellen, die ebenso wie die natürlichen
Stämme die weiter vorn genannten Verbindungen und Salze, insbesondere Dimethylphosphat, Monomethylphosphat oder Monomethylmethanphosphonat-Salze in wässriger Lösung abbauen können und Zellmasse produzieren. Solche Mutanten kann man chemisch, z.B. durch Behandlung mit gewissen Guanidinderivaten, z.B. N-Methyl-N'-nitro-N-nitrosoguanidin oder mit Alkalinitrit, z.B. Natriumnitrit, oder physikalisch, z.B. durch Ultraviolett-, Röntgen- oder radioaktive Strahlung, erzeugen.

Die erfindungsgemässen Mikroorganismen finden Anwendung in einem Verfahren zum mikrobiologischen Abbau in wässriger Phase von Phosphorsäuremethylestern, Mono- oder Diäthylphosphat, Methanphosphonsäuremethylestern und Salzen dieser Verbindungen, Methyl-, Dimethyl-, Trimethyl- oder Aethylammoniumsalzen oder Formiat- oder Acetatsalzen, sowie von Methanol oder Aethanol oder Mischungen dieser Verbindungen oder Salze, welches dadurch gekennzeichnet ist, dass man in wässriger Phase die weiter vorn genannten Mikroorganismen des Genus Hyphomicrobium, Mischkulturen dieser Mikroorganismen oder von diesen Mikroorganismen abgeleitete Mutanten in Gegenwart von essenziellen anorganischen Salzen und gegebenenfalls einer Stickstoffquelle bei ca. 20°C bis ca. 40°C und einem pH-Wert von ca. 4 bis ca. 7,5 unter aeroben Bedingungen züchtet und, wenn erwünscht, die erhältliche Zellmasse isoliert.

Die erfindungsgemässen Mikroorganismen, Mischkulturen oder Mutanten davon finden bevorzugt Anwendung in einem Verfahren zum mikrobiologischen Abbau in wässriger Phase von Trimethylphosphat, Dimethyl- oder Methylphosphatsalzen, z.B. den Natriumsalzen, Di-methyl- oder Methylmethanphosphonatsalzen, z.B. den Natriumsalzen.

Die erfindungsgemässen Mikroorganismen, Mischkulturen oder Mutanten davon finden in erster Linie Anwendung in einem Verfahren zum mikrobiologischen Abbau in wässriger Phase von Dimethylphosphat-, Methylphosphat- oder Methylmethanphosphonat-Salzen, insbesondere den Natriumsalzen.

Bei der Züchtung oder Fermentation bauen die in der Tabelle 1 aufgezählten Stämme die in wässriger Lösung, z.B. in einem Abwasser, befindlichen Verunreinigungen, z.B. Dimethylphosphat-, Methylphosphat- oder Methylmethanphosphonatsalze oder Mischungen dieser Salze, ab und verbrauchen Sauerstoff. Beim Abbauverfahren produzieren die Mikroorganismen Zellmasse, welche z.B. durch ein Vielfaches der ungefähren Summenformel $C_5H_9NO_x$ (x = 4-5) und einen Gehalt von ca. 37,0 % C, 6,2 % H und 9,6 % N (lyophilisiert) charakterisiert ist. Als weitere Fermentationsprodukte werden Kohlendioxid, beim Abbau der genannten Phosphorverbindungen ausserdem Phosphationen und beim Abbau der genannten Alkylammonium-Verbindungen ausserdem Ammoniumionen gebildet. Während der Fermentation ist der pH-Wert der zu reinigenden wässrigen Phase durch Zugabe von Pufferlösung, z.B. Phosphatpufferlösung, oder von wässrigen Basen, z.B. verdünnter, wässriger Natrium- oder Kaliumhydroxidlösung, auf Werte zwischen 4,0 und 7,5, bevorzugt ca. 6 bis 7, einzustellen. Die Zugabe der Säuren und Basen kann automatisch erfolgen.

Die gebildeten Phosphationen können mit Eisen(II)- oder Eisen(III)-Salzen, z.B. Ammoniumeisen(II)-sulfat, oder Kalkmilch (technische wässrige Calciumhydroxid-Lösung) ausgefällt und abgetrennt werden.

Die Züchtung erfolgt in Gegenwart von essenziellen anorganischen Salzen. Solche Salze sind beispielsweise in Wasser lösliche Halogenide, z.B. Chloride, Carbonate oder Sulfate von Alkalimetallen, z.B. Natrium oder Kalium, Erdalkalimetallen, z.B. Calcium oder Magnesium, oder Uebergangsmetallen, z.B. Eisen, Kobalt, Mangan, Molybdän, Kupfer oder Zink.

Bevorzugte essenzielle anorganische Salze sind beispielsweise die in der Nährlösung MV 7 enthaltenen Salze, z.B. Magnesium- und Eisensulfat und Kalium- und Calciumchlorid. In geringen Mengen können noch Zink-, Mangan- und Kupfersulfat, Natriummolybdat und Borax zugesetzt werden. Beim Abbau der genannten Alkylammonium-Verbindungen, der Formiat- oder Acetatsalze, sowie von Methanol oder Aethanol werden ausserdem Phosphatsalze, z.B. Dinatrium- oder
Dikaliumhydrogenphosphat und/oder Natrium- oder Kaliumdihydrogenphosphat zugesetzt.

Zur Reinigung von wässrigen Lösungen, welche beispielsweise ausschliesslich Dimethylphosphat-, Methylphosphat- oder Methylmethanphosphonatsalze und sonst keine Stickstoff-haltigen Verbindungen
enthalten, setzt man als Stickstoffquelle beispielsweise Aminosäuren, Alkylammoniumsalze, z.B. Methylammoniumchlorid, Ammoniumsalze, z.B. Ammoniumchlorid, oder Nitrate, z.B. Kalium- oder
Ammoniumnitrat, hinzu.

Die Züchtung erfolgt unter aeroben Bedingungen, z.B. unter Sauerstoff- oder Luftzufuhr, und zwar unter Schütteln oder Rühren in
Schüttelkolben oder Fermentern. Bei Verwendung der erfindungsgemässen Mikroorganismen in Kläranlagen kann die Züchtung in offenen
oder geschlossenen Klärbecken auch in Gegenwart von anderen Mikroorganismen erfolgen. So lassen sich bei Verwendung von Mischkulturen
enthaltend die weiter vorn genannten Mikroorganismen und beispielsweise die Mikroorganismen gemäss den Europäischen Patentanmeldungen 82114, 82814 oder 127581 wässrige Lösungen enthaltend die
weiter vorn genannten Verunreinigungen oder Bestandteile, z.B.
Dimethylphosphat-, Methylphosphat- oder Methylmethanphosphonatsalze, sowie als weitere Verunreinigungen beispielsweise Natriummethylsulfat, Trimethyläthylammoniumchlorid oder Dimethylformamid,
mikrobiologisch reinigen. Die Züchtung lässt sich in einem Temperaturbereich von ca. 25° bis ca. 35°C, bevorzugt bei ca. 27° bis
ca. 28°C, durchführen.

Die Züchtung kann ansatzweise, z.B. durch ein- oder mehrmalige
Zugabe von Nährlösung, oder kontinuierlich durch dauernde Zugabe von
Nährlösung erfolgen.

Vorzugsweise züchtet man in mehreren Stufen, indem man zunächst eine
oder mehrere Vorkulturen, z.B. in einem flüssigen Nährmedium, z.B.
Nährlösung MV 7, herstellt, welche man anschliessend in die eigentliche Hauptkultur überimpft. Eine Vorkultur lässt sich beispielsweise herstellen, indem man eine Probe mit Zellmaterial des betreffenden Mikroorganismus, der beispielsweise über Schrägagar aufbewahrt wird, in eine sterile Nährlösung, z.B. MV 7, mit einer
geeigneten Kohlenstoffquelle, z.B. Dimethylphosphat, überträgt und
mehrere Tage bei 28°C inkubiert. Mit dieser ersten Vorkultur impft
man frische Nährlösungen, z.B. MV 7, mit derselben Kohlenstoffquelle
an und inkubiert nochmals mehrere Tage bei 28°C.

Man kann den Fermentationsverlauf durch Probenentnahme analytisch
während der Fermentation verfolgen, z.B. durch Messung des pH-Wertes
der Kultur oder der optischen Dichte, welche ein Mass für das
Wachstum des jeweiligen Stamms ist, sowie gravimetrisch anhand des
Trockengewichts der gebildeten Zellmasse oder durch Bestimmung des
Phosphatgehalts beim Abbau der genannten Phosphorverbindungen.

Die Zellmasse ist ebenfalls Gegenstand der vorliegenden Erfindung.
Diese lässt sich z.B. nach einem der zahlreichen in der Europäischen
Patentschrift 0 010 243 beschriebenen Verfahren aufarbeiten und z.B.
in Düngemittel überführen. Die Zellmasse ist ein wertvoller Rohstoff, der eine definierte und reproduzierbare Zusammensetzung hat.
Ungefähre Summenformel: $C_5H_9NO_x$ (x = 4-5); Gehalt (Lyophilisat):
37,0 % C, 6,2 % H und 9,6 % N.

Ebenfalls Gegenstand der vorliegenden Erfindung sind die in der
Zellmasse enthaltenen Enzyme bzw. der aus der Zellmasse erhältliche
Enzymextrakt. Enzyme lassen sich aus der Zellmasse in an sich
bekannter Weise, z.B. wie in der Britischen Patentanmeldung 2,019,390 beschrieben, extrahieren.

Die verfahrensgemäss erhältliche Zellmasse kann als Einzellerprotein
beispielsweise als Viehfutterzusatz verwendet werden. Die Zellmasse
lässt sich auch beispielsweise als Suspension oder aufgearbeitet,
z.B. nach Entwässerung oder Pasteurisierung, als Düngemittel
verwenden. Man kann die Zellmasse auch als Ausgangsmaterial zur
Herstellung von Biogas mit hohem Heizwert (Zusammensetzung ca. 70 % Methan, 29 % Kohlendioxid und 1 % Wasserstoff, Heizwert
ca. 5500-6500 kcal/m$^3$), beispielsweise durch anaerobe Vergärung in
Faultürmen, verwenden. Der Rückstand (Faulschlamm) aus dem Herstellungsverfahren von Biogas ist ebenfalls hochwertiger Dünger, der im
Vergleich zur ursprünglichen Zellmasse mit Stickstoff stark angereichert ist.

Die folgenden Beispiele illustrieren die vorliegende Erfindung. Die
Temperaturangaben erfolgen in Grad Celsius.

Beispiel 1: (Herstellung der Vorkultur):
Eine Probe des auf Schrägagar aufbewahrten Mikroorganismus
DMPO/HW1-5 (NRRL-B-15645) wird in einen Schüttelkolben zu 20 ml
Nährlösung MV 7 mit der weiter vorn angegebenen Zusammensetzung
enthaltend Natriummethylmethanphosphonat oder Dimethylphosphat in
einer Konzentration von 5 g/l gegeben, und 72 Stunden bei 28° und
250 Upm inkubiert. 5-7 ml dieser ersten Vorkultur werden in einen
zweiten Schüttelkolben gegeben, enthaltend 100 ml Nährlösung MV 7
und Natriummethylmethanphosphonat oder Dimethylphosphat in einer
Konzentration von 5 g/l und 5 mMol Phosphatpuffer (pH 7), und
72 Stunden bei 28° und 250 Upm inkubiert.

Beispiel 2: Analog Beispiel 1 lassen sich Vorkulturen der Stämme
TMPO 1/8 (NRRL-B-15632), TMPO 1/9 (NRRL-B-15633), TMPO 1/10
(NRRL-B-15634), TMPO 2/3 (NRRL-B-15635), TMPO 2/6 (NRRL-B-15636),
TMPO 2/9 (NRRL-B-15637), TMPO 2/10 (NRRL-B-15638), TMPO 2/13
(NRRL-B-15639), DMMP 1/6 (NRRL-B-15640), DMMP 1/9 (NRRL-B-15641),
DMMP 2/10 (NRRL-B-15642), DMMP 2/11 (NRRL-B-15643), DMMP 2/15
(NRRL-B-15644) und DMPO/WH1-5 (NRRL-B-15645) züchten.

Beispiel 3: In einem Laborfermenter werden gegebenenfalls hitzesterilisierte (Sterilisation 20 Min. bei 120°) Nährlösung MV 7 mit
einer gegebenenfalls sterilfiltrierten, wässrigen Natriummethylmethanphosphonat-Lösung so vereinigt, dass ein ungefähres Arbeitsvolumen von 10 l mit einer Konzentration von ca. 5 g/l Natriummethylmethanphosphonat resultiert.

Man gibt eine Probe mit ca. 500 ml Vorkultur des Stamms DMPO/HW1-5
(NRRL-B-15645) hinzu und hält im Fermenter folgende Bedingungen ein:
pH-Wert von 7,0, welcher durch Zugabe jeweils von wässriger 0,1 N
NaOH- bzw. 0,1 N HCl-Lösung konstant gehalten wird, Temperatur: 28°,
Luftzufuhr 0,25 l/l Min. und Rührgeschwindigkeit von 400-700 Upm.

Der Stamm wächst in dieser Lösung enthaltend Natriummethylmethanphosphonat als einziger Kohlenstoffquelle. Nach ca. 90 Stunden ist
das eingesetzte Natriummethylmethanphosphonat zu mehr als 95 %
abgebaut. Die gebildete Zellmasse liegt als Gemisch von Einzelzellen
oder Aggregaten verschiedener Grösse vor, welche man durch Absetzen
oder Zentrifugieren abtrennen kann. Die gebildeten Phosphationen
können mit Eisen(II)- oder Eisen(III)-salzen, z.B. Ammonium-
eisen(II)-sulfat, oder wässriger Calciumhydroxidlösung ausgefällt
und abgetrennt werden.

Beispiel 4: Analog Beispiel 3 lassen sich in einem Laborfermenter
Natriumdimethylphosphat oder Dinatriummethylphosphat in wässriger
Lösung zu mehr als 95 % abbauen, indem man die wässrige Lösung mit
Vorkulturen des Stamms DMPO/HW1-5 (NRRL-B-15645 beimpft und im
Fermenter die im Beispiel 3 genannten Bedingungen einhält.

Beispiel 5: Analog Beispiel 3 lassen sich in einem Laborfermenter
Natriummethylmethanphosphonat, Natriumdimethylphosphat oder
Dinatriummethylphosphat in wässriger Lösung zu mehr als 95 %
abbauen, indem man die wässrige Lösung  mit Vorkulturen der Stämme:
TMPO 1/8 (NRRL-B-15632), TMPO 1/9 (NRRL-B-15633), TMPO 1/10
(NRRL-B-15634), TMPO 2/3 (NRRL-B-15635), TMPO 2/6 (NRRL-B-15636),
TMPO 2/9 (NRRL-B-15637), TMPO 2/10 (NRRL-B-15638), TMPO 2/13
(NRRL-B-15639), DMMP 1/6 (NRRL-B-15640), DMMP 1/9 (NRRL-B-15641),
DMMP 2/10 (NRRL-B-15642), DMMP 2/11 (NRRL-B-15643), DMMP 2/15
(NRRL-B-15644) und DMPO/HW1-5 (NRRL-B-15645)  beimpft und im
Fermenter die im Beispiel 3 genannten Bedingungen einhält.

Beispiel 6: Analog Beispiel 3 lassen sich industrielle Abwässer
(Mutterlaugen) enthaltend beispielsweise ca. 5 - 7 % Dimethylphosphat, ca. 4 -6 % Dimethylformamid, ca. 1 - 2 % Methanol, ca 1 %
NaCl, ca. 1 % Natriumsulfat und ca. 70 % Wasser (in einer Konzentration von ca. 100 ml Abwasserlösung pro Liter Arbeitsvolumen),
mikrobiologisch reinigen, indem man einen Fermenter oder Klärbecken
(offen oder geschlossen) mit Vorkulturen des Stamms DMPO/HW1-5 und
DMF 5/8 (NRRL-B-15 368), siehe Europäische Patentanmeldung
Nr. 127581, beimpft und die in Beispiel 3 genannten Bedingungen
einhält. Man erhält eine wässrige Phase enthaltend Zellmasse sowie
gelöstes Natriumphosphat, Natriumchlorid und Natriumsulfat. Die
Zellmasse und das gelöste Natriumphosphat lassen sich auf die in
Beispiel 3 geschilderte Weise abtrennen. Bei Verwendung von wässriger Calciumhydroxid-Lösung werden die Phosphationen als Calciumphosphat und die Sulfationen als Calciumsulfat ausgefällt.

<u>Patentansprüche</u>

1. Mikroorganismen des Genus Hyphomicrobium, Mischkulturen oder
Mutanten davon, dadurch gekennzeichnet, dass sie in wässriger Phase
Phosphorsäuremethylester, Mono- oder Diäthylphosphat, Methanphosphonsäuremethylester oder Salze dieser Verbindungen, Methyl-,
Dimethyl-, Trimethyl- oder Aethylammoniumsalze oder Formiat- oder
Acetatsalze sowie Methanol oder Aethanol oder Mischungen dieser
Verbindungen abbauen und Zellmasse produzieren.

2. Mikroorganismen des Genus Hyphomicrobium gemäss Anspruch 1,
Mischkulturen oder Mutanten davon, dadurch gekennzeichnet, dass sie
in wässriger Phase Trimethylphosphat, Dimethyl- oder Methylphosphatsalze, Dimethylmethanphosphonat oder Methylmethanphosphonatsalze
oder Mischungen dieser Verbindungen oder Salze abbauen und Zellmasse produzieren.

3. Mikroorganismen des Genus Hyphomicrobium gemäss Anspruch 1,
Mischkulturen oder Mutanten davon, dadurch gekennzeichnet, dass sie
in wässriger Phase Dimethylphosphat-, Methylphosphat- oder Methylmethanphosphonatsalze oder Mischungen dieser Verbindungen oder Salze
abbauen und Zellmasse produzieren.

4. Mikroorganismen des Genus Hyphomicrobium gemäss Anspruch 1 aus
der Gruppe folgender Stämme: TMPO 1/8 (NRRL-B-15632),
TMPO 1/9 (NRRL-B-15633), TMPO 1/10, (NRRL-B-15634), TMPO 2/3
(NRRL-B-15635), TMPO 2/6 (NRRL-B-15636), TMPO 2/9 (NRRL-B-15637),
TMPO 2/10 (NRRL-B-15638), TMPO 2/13 (NRRL-B-15639), DMMP 1/6
(NRRL-B-15640), DMMP 1/9 (NRRL-B-15641), DMMP 2/10 (NRRL-B-15642),
DMMP 2/11 (NRRL-B-15643), DMMP 2/15 (NRRL-B-15644) und DMPO/HW1-5
(NRRL-B-15645), Mischkulturen oder Mutanten davon.

5. Verfahren zum mikrobiologischen Abbau in wässriger Phase von
Phosphorsäuremethylestern, Mono- oder Diäthylphosphat, Methanphosphonsäuremethylestern und Salzen dieser Verbindungen, Methyl-, Dimethyl-, Trimethyl- oder Aethylammoniumsalzen oder Formiat- oder
Acetatsalzen sowie von Methanol oder Aethanol oder Mischungen
dieser Verbindungen oder Salze, dadurch gekennzeichnet, dass man in
wässriger Phase, Mikroorganismen des Genus Hyphomicrobium gemäss
Anspruch 1, Mischkulturen dieser Mikroorganismen oder von diesen
Mikroorganismen abgeleitete Mutanten, in Gegenwart von essenziellen
anorganischen Salzen und gegebenenfalls einer Stickstoffquelle bei
ca. 20°C bis ca. 40°C und einem pH-Wert von ca. 4 bis ca. 7,5 unter
aeroben Bedingungen züchtet und, wenn erwünscht, die erhältliche
Zellmasse isoliert.

6. Verfahren gemäss Anspruch 5 zum mikrobiologischen Abbau in
wässriger Phase von Trimethylphosphat, Dimethyl- oder Methylphosphatsalzen, Dimethylmethanphosphonat oder Methylmethanphosphonatsalzen oder Mischungen dieser Verbindungen oder Salze,
dadurch gekennzeichnet, dass man in wässriger Phase Mikroorganismen
aus der Gruppe folgender Stämme: TMPO 1/8 (NRRL-B-15632), TMPO 1/9
(NRRL-B-15633), TMPO 1/10 (NRRL-B-15634), TMPO 2/3 (NRRL-B-15635),
TMPO 2/6 (NRRL-B-15636), TMPO 2/9 (NRRL-B-15637), TMPO 2/10
(NRRL-B-15638), TMPO 2/13 (NRRL-B-15639), DMMP 1/6 (NRRL-B-15640),
DMMP 1/9 (NRRL-B-15641), DMMP 2/10 (NRRL-B-15642), DMMP 2/11
(NRRL-B-15643), DMMP 2/15 (NRRL-B-15644) und DMPO/HW1-5
(NRRL-B-15645), Mischkulturen oder Mutanten davon züchtet.

7. Verfahren gemäss Anspruch 5 zum mikrobiologischen Abbau in
wässriger Phase von Dimethylphosphat-, Methylphosphat- oder Methylmethanphosphonatsalzen oder Mischungen davon, dadurch gekennzeichnet, dass man Mikroorganismen aus der Gruppe folgender
Stämme: TMPO 1/8 (NRRL-B-15632), TMPO 1/9 (NRRL-B-15633), TMPO 1/10
(NRRL-B-15634), TMPO 2/3 (NRRL-B-15635), TMPO 2/6 (NRRL-B-15636),
TMPO 2/9 (NRRL-B-15637), TMPO 2/10 (NRRL-B-15638), TMPO 2/13
(NRRL-B-15639), DMMP 1/6 (NRRL-B-15640), DMMP 1/9 (NRRL-B-15641),
DMMP 2/10 (NRRL-B-15642), DMMP 2/11 (NRRL-B-15643), DMMP 2/15
(NRRL-B-15644) und DMPO/HW1-5 (NRRL-B-15645), Mischkulturen oder
Mutanten davon züchtet.

8. Verfahren nach einem der Ansprüche 5-7, dadurch gekennzeichnet, dass man die Züchtung bei 28°C durchführt.

9. Verfahren nach einem der Ansprüche 5-8, dadurch gekennzeichnet, dass man die Züchtung kontinuierlich durchführt.

10. Die nach dem Verfahren gemäss Anspruch 5 erhältliche Zellmasse.

11. Zellmasse gemäss Anspruch 10, gekennzeichnet durch ein Vielfaches der ungefähren Summenformel $C_5H_9NO_x$ (x = 4-5) und einen Gehalt (Lyophilisat) von 37,0 % C, 6,2 % H und 9,6 % N.

12. Die in der Zellmasse gemäss Anspruch 11 enthaltenen Enzyme.

13. Verwendung der Zellmasse gemäss Anspruch 11 als Düngemittel.

FO 7.4 RS/eg*